# EUROPÄISCHE PATENTANMELDUNG

(11) **EP 2 023 234 A1**
(43) Veröffentlichungstag der Anmeldung: **11.02.2009**
(21) Anmeldenummer: 08011107.3
(22) Anmeldetag: 19.06.2008
(51) Int. Cl.: G06F 3/033

(54) **Digitales Röntgenbildbetrachtungsgerät für die medizinische Diagnostik**

(30) Priorität: 09.08.2007 DE 202007011152 U
(71) Anmelder: S-CAPE GmbH, 08468 Reichenbach (DE)
(72) Erfinder: Seidel, Uwe, 08468 Reichenbach (DE)
(74) Vertreter: Auerbach, Bettina

(57) **Zusammenfassung**

Die Erfindung betrifft ein digitales Röntgenbildbetrachtungsgerät für die medizinische Diagnostik, bestehend aus einem klinischen Raumbedingungen angepasstem Gehäuse, wenigstens einem Bildschirm, wobei dem Röntgenbildbetrachtungsgerät ein den Wechsel der zugänglichen und anzeigbaren Bild- und Textinformationen realisierender berührungslos bedienbarer Sensor zugeordnet ist.

## Beschreibung

Die Erfindung betrifft ein digitales Röntgenbildbetrachtungsgerät für die medizinische Diagnostik, welches aus einem klinischen Raumbedingungen angepasstem Gehäuse und wenigstens einem Bildschirm ausgestattet ist. Anwendung finden derartige Lösungen vor allem in Krankenhäusern und dort vor allem in Operationsräumen.

Vorrichtungen für die Röntgenbildbetrachtung im medizinischen Diagnostikbereich sind bereits in vielfältiger Weise bekannt. All diese Lösungen tragen jeweils speziellen Anforderungen des medizinischen oder klinischen Anwendungsbereich in ihrer Weise Rechnung.

Der erfindungsgemäßen Lösung liegt deshalb die Aufgabe zugrunde, eine Möglichkeit zu schaffen, ein digitales Röntgenbildbetrachtungsgerät berührungslos bedienen zu können, so dass es dem Benutzer möglich ist, auf den Inhalt der im Röntgenbilderfassungsgerät enthaltenen digitalen Informationen zugreifen zu können, ohne den Status der erforderlichen Sterilität ihrer Hände verzichten zu müssen.

Die Lösung soll sich des weiteren durch eine Bedienfreundlichkeit auszeichnen und auf die jeweiligen Anforderungen des Benutzers einstellbar sein.

Erfindungsgemäß wird diese Aufgabe durch die Merkmale des Anspruchs 1 gelöst.

Danach besteht ein digitales Röntgenbildbetrachtungsgerät für die medizinische Diagnostik aus einem klinischen Raumbedingungen angepasstem Gehäuse, und wenigstens einem Bildschirm sowie wenigstens einem den berührungslosen Wechsel der zugänglichen und anzeigbaren Bild- und Textinformationen realisierenden Sensor.

Vorteilhafter Weise ist der berührungslos bedienbare Sensor im Röntgenbildbetrachtungsgerät angeordnet. Es ist auch möglich, diesen Sensor als ein dem Röntgenbildbetrachtungsgerät zugehöriges externes Bedienfeld auszubilden.

Ist der Sensor im Röntgenbildbetrachtungsgerät selbst integriert, besteht die Möglichkeit, diesen hinter dem Bildschirmschutzglas oder innerhalb des Gehäuses anzuordnen, wobei seine genaue Lage durch ein Symbol kenntlich gemacht ist.

Um zu vermeiden, dass ungewünschte Veränderungen der Bildschirmanzeige zur Bewegungen der Personen hervorgerufen werden, ist es von Vorteil, den berührungslos bedienbare Sensor einen separaten ebenfalls berührungslos bedienbaren Ein-/Aus-Schalter zuzuordnen.

Der berührungslos bedienbare Sensor kann als optischer Sensor, als auf Temperaturveränderungen reagierender Sensor, als kapazitiver oder induktiver Sensor ausgebildet sein, wobei auch andersartig funktionierende Sensor zum Einsatz kommen können.

Ebenfalls sollte der berührungslos bedienbare Sensor mit einem Zoom oder der Möglichkeit der Bildverschiebung ausgestattet sein, um dem Benutzer zu ermöglichen, Bildausschnitte näher betrachten zu können.

Um die Benutzerfreundlichkeit des Sensors zu erhöhen ist es auch möglich, den berührungslos bedienbare Sensor mit einem die Geschwindigkeit der Bedienung regulierendem Baustein auszustatten.

Um diese verschiedenen Funktionen mit einem Sensor ausführen zu können, ist der Sensor in mehrere den verschiedenen Funktionen zugeordnete Erfassungsbereiche unterteilt.

Es ist auch möglich, den Sensor aus Sensoreinheit auszubilden, die aus mehreren Sensoren besteht. Dann können den einzelnen Sensoren jeweilige Funktionen zugeordnet werden.

Die einzelnen Funktionsbereiche sind von Außen über Symbolanordnungen sichtbar gemacht.

Ein mit dieser erfindungsgemäßen Lösung ausgestattetes Röntgenbilderfassungsgerät erlaubt den Einsatz in einem Operationsraum und kann vom OP-Team selbst betätigt werden, ohne dass diese den Status der Sterilität ihrer Hände verlieren.

Desweiteren ist es möglich, das Gerät auf die jeweiligen Anforderungen des Bedieners einzustellen.

Die Erfindung soll nachstehend an Ausführungsbeispielen näher erläutert werden.

### Ausführungsbeispiele

### Ausführungsbeispiel 1:

Ein an der Deckenampel über einem Operationstisch angeordnetes Röntgenbildbetrachtungsgerät enthält digitale Bildinformationen einer MRT-Aufzeichnung zum Krankheitsbild des Patienten.

Zum Verändern der Ansichtsanzeige weist das Röntgenbildbetrachtungsgerät einen im Gerät selbst und dort hinter dem Bildschirmschutzglas angeordneten Sensor auf, welcher in diesem Fall als kapazitiver Sensor ausgebildet ist und durch eine Handbewegung vor dem Bildschirm von dem Benutzer bedient werden kann. Dieser Sensor steht in Verbindung mit dem Rechnerbaustein und aktiviert so das Weiterblättern der Bildinformationen auf dem Bildschirm. Da der Sensor mit mehreren Funktionen ausgestattet ist, ist er als Sensoreinheit ausgebildet und besteht aus mehreren Sensoren, denen unterschiedliche Funktionen zugeordnet sind und deshalb jeweils separat positioniert sind. Deren Lage und Funktion ist durch Symbole auf dem Bildschirm angezeigt. So ist es möglich, die berührungslos bedienbare Sensoreinheit zu aktivieren oder deaktivieren, in dem vorhandenen Bildmaterial zu blättern und Ausschnitte zu vergrößern oder zu verschieben. Es ist auch möglich, die Bedieneinheit auf die vom Benutzer jeweils gewünschte Reaktionsgeschwindigkeit einzustellen.

### Ausführungsbeispiel 2:

Für spezielle Operationen kann es auch notwendig werden, dass der berührungslose Sensor bzw. die Sensoreinheit in einem zum Röntgenbildbetrachtungsgerät zugehörigen jedoch extern angeordneten Bedienfeld untergebracht ist. Auch hier sind die Lage und die Funktionen der einzelnen Sensoren von Außen sichtbar gemacht und werden über Bewegungen vor dem Sensor bedient.

Mit dieser Lösung kann sich der Benutzer ohne Berührung des Bildschirms oder einem externen Bedienfeld die gewünschten Ansichten des Bildmaterials oder auch von Textpassagen auf den Bildschirm holen. Die an einer Operation beteiligten Akteure bleiben flexibel und dennoch steril. Das Bedienfeld kann dort positioniert werden, wo es der Benutzer wünscht.

## Patentansprüche

1. Digitales Röntgenbildbetrachtungsgerät für die medizinische Diagnostik, bestehend aus einem klinischen Raumbedingungen angepasstem Gehäuse, wenigstens einem Bildschirm, **dadurch gekennzeichnet, dass** an dem Röntgenbildbetrachtungsgerät ein den Wechsel der zugänglichen und anzeigbaren Bild- und Textinformationen realisierender berührungslos bedienbarer Sensor angeordnet ist.

2. Digitales Röntgenbildbetrachtungsgerät für die medizinische Diagnostik nach dem Anspruch 1, **dadurch gekennzeichnet, dass** der berührungslos bedienbare Sensor im Röntgenbildbetrachtungsgerät angeordnet ist.

3. Digitales Röntgenbildbetrachtungsgerät für die medizinische Diagnostik nach dem Anspruch 1, **dadurch gekennzeichnet, dass** der berührungslos bedienbare Sensor in einem dem Röntgenbildbetrachtungsgerät zugehörigem externen Bedienfeld angeordnet ist.

4. Digitales Röntgenbildbetrachtungsgerät für die medizinische Diagnostik nach einem der Ansprüche 1 und 2, **dadurch gekennzeichnet, dass** der berührungslos bedienbare Sensor hinter dem Bildschirmschutzglas angeordnet ist.

5. Digitales Röntgenbildbetrachtungsgerät für die medizinische Diagnostik nach einem der Ansprüche 1 und 2, **dadurch gekennzeichnet, dass** der berührungslos bedienbare Sensor innerhalb des Gehäuses angeordnet ist.

6. Digitales Röntgenbildbetrachtungsgerät für die medizinische Diagnostik nach einem der Ansprüche 1 bis 5, **dadurch gekennzeichnet, dass** dem berührungslos bedienbare Sensor ein separater Ein-/Aus-Aktivator zugeordnet ist.

7. Digitales Röntgenbildbetrachtungsgerät für die medizinische Diagnostik nach einem der Ansprüche 1 bis 6, **dadurch gekennzeichnet, dass** der berührungslos bedienbare Sensor als optischer Sensor, als Temperatursensor, als kapazitiver oder als induktiver Sensor ausgebildet ist.

8. Digitales Röntgenbildbetrachtungsgerät für die medizinische Diagnostik nach einem der Ansprüche 1 bis 7, **dadurch gekennzeichnet, dass** der berührungslos bedienbare Sensor mit einem ebenfalls berührungslos bedienbaren Zoom und der Funktionsmöglichkeit der Bildverschiebung ausgestattet ist.

9. Digitales Röntgenbildbetrachtungsgerät für die medizinische Diagnostik nach einem der Ansprüche 1 bis 8, **dadurch gekennzeichnet, dass** der berührungslos bedienbare Sensor mit einem die Geschwindigkeit der Bedienung regulierendem Baustein ausgestattet ist.

10. Digitales Röntgenbildbetrachtungsgerät für die medizinische Diagnostik nach einem der Ansprüche 1 bis 9, **dadurch gekennzeichnet, dass** der berührungslos bedienbare Sensor in mehrere unterschiedliche Funktionen regelnde Erfassungsbereiche unterteilt ist.

11. Digitales Röntgenbildbetrachtungsgerät für die medizinische Diagnostik nach einem der Ansprüche 1 bis 9, **dadurch gekennzeichnet, dass** der berührungslos bedienbare Sensor als Sensoreinheit, bestehend aus mehreren und verschiedenen Funktionen zugeordneten Sensoren, ausgebildet ist.
